# EUROPEAN PATENT SPECIFICATION

(11) **EP 3 494 798 B1**
(45) Date of publication and mention of the grant of the patent: **11.01.2023**
(21) Application number: 17837219.9
(22) Date of filing: 01.08.2017
(51) Int. Cl.: A23L 3/01, A23L 3/015, A23L 3/3472, A23L 3/3463, A23B 4/005, B65B 55/14, A61L 2/04, A61L 2/08, B65B 55/02, A23B 4/01, A23B 7/005, A23B 7/01, A61L 2/12

(54) **METHOD FOR STERILIZING PROCESSED FOODS COMPRISING MICROWAVE HEATING PRETREATMENT**
VERFAHREN ZUR STERILISATION VON VERARBEITETEN LEBENSMITTELN MIT MIKROWELLENVORBEHANDLUNG
PROCÉDÉ DE STÉRILISATION D'ALIMENTS TRANSFORMÉS COMPRENANT UN PRÉTRAITEMENT DE CHAUFFAGE PAR MICRO-ONDES

(30) Priority: 05.08.2016 KR 20160099998
(43) Date of publication of application: 12.06.2019
(73) Proprietor: CJ Cheiljedang Corporation, Seoul 04560 (KR)
(72) Inventor: CHO, Won Il, Seoul 08018 (KR); CHOI, Su Hee, Suwon-si Gyeonggi-do 16507 (KR); PARK, Hee Joon, Seoul 01370 (KR); YOON, Sang Eun, Suwon-si Gyeonggi-do 16508 (KR); LEE, Nam Ju, Seoul 06592 (KR); LEE, Jong Il, Seoul 08324 (KR)
(74) Representative: Millburn, Julie Elizabeth
(86) International application number: PCT/KR2017/008289
(87) International publication number: WO 2018/026168

(56) References cited:
- WO-A1-2016/044571
- CN-A- 102 069 923
- CN-A- 105 685 762
- CN-U- 201 663 897
- JP-A- S5 843 773
- JP-A- H09 163 961
- JP-A- S61 257 148
- JP-A- 2010 022 364
- KR-A- 20160 002 159
- KR-A- 20160 002 159
- KR-A- 20160 078 098
- KR-B1- 101 636 626
- HARLFINGER L: "MICROWAVE STERILIZATION", FOOD TECHNOLOGY, INSTITUTE OF FOOD TECHNOLOGISTS, CHICAGO, IL, US, vol. 46, no. 12, 1 December 1992 (1992-12-01), pages 57-61, XP000331049, ISSN: 0015-6639

## Description

### Technical Field

The present application relates to a sterilization method, which uses a retort sterilization method comprising microwave heating pretreatment, thereby maintaining the intrinsic texture of vegetables, meat, or seafood and flavors of aromatic vegetables such as red pepper, garlic, ginger, etc. compared to conventional sterilization methods, and effectively killing fungi, yeast, pathogenic bacteria, and sporogenic heat-resistant microorganisms.

### BACKGROUND ART

Recently, according to the lifestyle of modern people, there is an increasing demand for convenient processed foods such as foods for microwave cooking. Representative examples of convenience foods are retort sterilized room temperature distribution pouches and tray products. In order to mass-produce these products, it is necessary to apply various processing processes such as raw material pretreatment, seasoning, cooking, sterilization, packaging, etc. The establishment of optimization conditions for these processes is the most important process in ensuring excellent quality and microbial safety.

The main raw materials for these convenience foods are aromatic vegetables such as garlic and onion, and root vegetables such as potato and carrot. These raw materials are rich in nutrient cells and spores of Bacillus strains originated from the soil and therefore, microbial safety is greatly deteriorated.

In particular, contamination by heat-resistant spore-type Bacillus microorganisms causes organoleptic quality deterioration due to sour taste, and in severe cases, causes gas generation and swelling. Therefore, for commercialization, the design and application of appropriate sterilization processes should be considered first.

In general, a retort sterilization method in which after sealing, heat is applied at the high temperature of 121°C and high pressure for several tens of minutes, is commercially applied, in order to kill heat-resistant spores. With the retort sterilization method, the heat-resistant spores may be killed, but the organoleptic qualities such as taste, appearance, and texture of an original ingredient are severely damaged by the high temperature, and many nutritive components are also destroyed. Therefore, there are many limitations in commercialization of various high quality processed foods.

Meanwhile, the mechanism of microwave sterilization has been identified to be microorganism death mainly due to high frequency-induced rapid heat generation of food which acts as a dielectric. The non-thermal sterilization effect of the electromagnetic field on microorganisms has not been clearly investigated yet. Most researches on sterilization of processed foods using microwave are related to the inactivation of pathogenic and spoilage microorganisms and enzymes at 50 to 82°C by pasteurization which is applicable to most refrigerated products. However, detailed researches on energy use, initial temperatures of food to be treated, physical properties of food and packaging materials, passage rate, which are important influence factors in sterilization have not been carried out much.

Currently commercialized processing and sterilization processes could neither effectively control heat-resistant microorganisms in various vegetable raw materials nor preserve the organoleptic quality and nutritive components of an original ingredient, and therefore, a new sterilization method capable of effectively controlling microorganisms while preserving the quality has been required.

A number of prior art methods are known from the following prior art.

KR20160078098 discloses a method for producing an instant cooking food, comprising a process of respectively separating and sterilizing solids and meat broth. An instant cooking broth product is produced through a process of separately sterilizing solids and meat broth and a process of preheating the solids through microwaves. Accordingly, compared to a conventional single process (a process of simultaneously sterilizing solids and meat broth), heating time is effectively reduced, so a food is prevented from being denatured by heat and process costs can be minimized.

CN105685762 discloses a preservation method of fresh wet raw noodles, comprising: bagging, microwave sterilizing, sealing when sterilized noodles are hot, sterilizing by water bath, and cooling. The bagging is performed when the noodles are hot after microwave heating, and the problems that microwave heating in the prior art easily causes bag expansion and breakage are effectively solved.

CN102069923 discloses a method for producing fresh-keeping instant wet noodles, which comprises steps of noodle making, bagging, microwave sterilization, aseptic sealing packaging, heat preservation sterilization, cooling and drying. The method is characterized in that the bagging comprises: weighing the wet noodles, filling the wet noodles into packaging bags and pre-sealing the packaging bags in a way that openings of the packaging bags are incompletely sealed. The aseptic packaging sealing comprises wet noodles flowing out of a delivery outlet of a microwave oven, entering an aseptic sealing packaging equipment, and being completely sealed in an aseptic environment after reaching a section of channel for finishing the sealing.

### DISCLOSURE OF THE INVENTION

### TECHNICAL PROBLEM

An aspect of the present application provides a sterilization method capable of preserving the organoleptic quality and nutritive components of an original ingredient and effectively controlling microorganisms.

### TECHNICAL SOLUTION

According to an aspect of the present application, there is provided a sterilization method comprising: 1) a pretreating microbial reduction step for microwave heat-treating an original ingredient; 2) a packaging step for packaging the pretreated original ingredient; and 3) a sterilization step for retort sterilizing the packaged original ingredient, wherein the original ingredient in step 1) is an original ingredient having been immersed in a natural antimicrobial solution; wherein in step 2), the original ingredient is packaged together with sauce or broth; and wherein the retort sterilization in step 3) is performed for 20 to 40 minutes at 110 to 115°C, or the retort sterilization in step 3) is performed for 5 to 15 minutes at 121 to 125°C. The invention is defined by the appended set of claims.

### ADVANTAGEOUS EFFECTS

The sterilization method of the present application minimizes the heat-induced deterioration of qualities, such as inherent taste, flavor and texture, in various convenient processed foods such as substitute foods, side dishes, dishes, in which vegetables, meats, and seafood are used as raw materials, and therefore, allows the convenient processed foods to have the intrinsic textures of vegetable, meat and seafood and the flavors of aromatic vegetables, such as red pepper, garlic, ginger, etc. remain and can effectively control fungi, yeast, pathogenic bacteria, and sporogenic heat-resistant microorganisms associated with hygiene and quality deterioration in raw materials.

### BRIEF DESCRIPTION OF THE DRAWINGS

FIG. 1 shows a flow chart of a processed food sterilization method of the present application.
Fig. 2 shows the experimental result of Example 2 (1) that is microwave heating and mild retort combination sterilization against the total bacteria, heat-resistant bacteria and fungi in vegetables.

### EXAMPLE EMBODIMENTS

Hereinafter, the present application will be described in detail.

Step 1) of the present application is a pretreating microbial reduction step for microwave heat-treating an original ingredient, and the original ingredient may be, but is not limited to, vegetables, meats, and seafood used for foods. The original ingredient may be prepared by selecting, washing and cutting thereof before the microbial reduction step of the present application. Specifically, preparation may be carried out by selecting an unimpaired original ingredient, washing the original ingredient with water several times, in order to remove contaminants such as dirt, foreign substances, etc. attached to the surface of the original ingredient, and then, cutting the same to a suitable size for each product. The pretreating microbial reduction may be carried out by microwave heat-treating the thus prepared original ingredient.

The microbial reduction by microwave heat-treatment has been identified to be microorganism death due to high frequency-induced rapid heat generation of food which acts as a dielectric. The microwave-induced heating results from dielectric loss generated by dipoles and polarization by an electromagnetic wave having a frequency of 300 MHz to 300 GHz and a wavelength of 1 mm to 1 m. In the case of foods, the microwave-induced vibration energy of water molecules causes rapid heat generation, which results in rapid drying speed, moisture homogenization, surface hardening, and a crack-preventive effect, a bleaching effect, and a sterilizing effect, and thus, the microwaves are currently used for sterilization, thawing, cooking, swelling, etc. In particular, the microwave has been widely used as a new sterilization method due to various advantages, such as sterilization of microorganisms and pests, extension of shelf life, improvement of quality by sterilization time shortening, possibility of sterilizing after packaging, etc. recently.

The microwave may be generated by a known microwave generator. Preferably, a continuous or batch microwave heating apparatus may be used, but the present application is not limited thereto. The microwave may be a microwave having an output of 700 to 1,200 W. If the microwave output is less than 700 W, the microbial reduction effect is insignificant, and if the microwave output is more than 1,200 W, the quality is deteriorated due to excessive heating.

In order to improve the microbial reduction effect of step 1), the microwave heat-treatment may be carried out under pressurized condition. Specifically, the microwave heat-treatment may be carried out under the condition of 133.322 -226.6481 kPa (1,000 to 1,700 mmHg). If the applied pressure is less than 133.322 kpa (1,000 mmHg) it is difficult to expect improvement of the microbial reduction effect. If the applied pressure is more than 226.6481 kPa (1,700 mmHg) the process may be costly and the quality of the original ingredient may deteriorate.

Step 1) may be carried out for 1 to 30 minutes. Preferably, step 1) may be carried out for 2 to 10 minutes. If the microwave heat-treatment is carried out for less than 1 minute, it is difficult to obtain the pretreating microbial reduction effect. If the microwave heat-treatment is carried out for more than 30 minutes, the original ingredient is excessively heated and the quality of the original ingredient may deteriorate after the overall sterilization.

Step 2) of the present application is a step of packaging the pretreated original ingredient. A packaging material for packaging the pretreated original ingredient may be a known packaging material, and preferably a tray or pouch may be used, but the present application is not limited thereto. A packaging apparatus may be a known apparatus, and preferably an automatic rotary packer or a tray automatic packing machine may be used, but the present application is not limited thereto. In one example of step 2), the pretreated original ingredient may be put into a pouch or a tray, and a sauce or broth may be put into the pouch or tray, and then the pouch or tray may be sealed and packaged.

Step 3) of the present application is a step of retort sterilizing the packaged original ingredient. Conventional retort sterilization is carried out by heating at a high temperature of 121°C and a high pressure for 15 to 20 minutes after sealing, so that the heat-resistant spores may be killed, but the quality of the original ingredient is deteriorated by the long time high temperature. However, the present application comprises the pretreating microbial reduction step of microwave heat-treatment, and optionally uses the original ingredient treated with a natural antimicrobial solution, and thus, with even milder retort sterilization than conventional retort sterilization, the same microbial reduction effect may be obtained as the sterilization effects of conventional sterilization methods. Step 3) may be carried out by a known apparatus, and preferably, the sterilization may be carried out by hydrothermal or spray retort equipment, but the present application is not limited thereto. In addition, step 3) may be carried out under mild conditions compared to conventional retort sterilization. Preferably, low temperature retort sterilization of heating at 110 to 115°C for 20 to 40 minutes or short time retort sterilization of heating at 121 to 125°C for 5 to 15 minutes, preferably for 7 to 12 minutes may be carried out. After step 3), a step of drying the surface of a product may be included for processing convenience.

The sterilization method of the present application minimizes the heat-induced deterioration of qualities, such as inherent taste, flavor and texture, in various convenient processed foods such as substitute foods, side dishes, dishes, in which vegetables, meats, and seafood are used as raw materials, compared to conventional retort sterilization methods, and therefore, allows the convenient processed foods to have the intrinsic textures of vegetable, meat and seafood and the flavors of aromatic vegetables, such as red pepper, garlic, ginger, etc. remain and can effectively sterilize fungi, yeast, pathogenic bacteria, and sporogenic heat-resistant microorganisms associated with hygiene and quality deterioration in raw materials.

In the sterilization method of the present application, the original ingredient in step 1) is an original ingredient having been immersed in a natural antimicrobial solution. The immersion treatment in the natural antimicrobial solution prior to the pretreating microbial reduction may reduce the count of initial microorganisms in the original ingredient, and thus, may enhance the microbial reduction effect in the subsequent pretreating microbial reduction step and the sterilization effect in the mild retort process. The natural antimicrobial solution may comprise any one or more natural antimicrobials selected from organic acids, surfactants, bacteriocin, and calcium preparations. The organic acid may be at least one of lactic acid, citric acid, phytic acid, malic acid, acetic acid, and succinic acid.

The natural antimicrobial solution may contain 0.01 to 3.0 weight% of a natural antimicrobial and the original ingredient may be immersed therein for 10 to 120 minutes. If the amount of the natural antimicrobial is less than 0.01 weight% or the immersion time is less than 10 minutes, it is difficult to obtain the effect of reducing the initial microorganism count in the original ingredient. If the amount of the natural antimicrobial is more than 3.0 weight% or the immersion time is more than 120 minutes, the organoleptic function of the original ingredient may be affected and the quality may be deteriorated.

As one example of the immersion treatment in the natural antimicrobial solution, the original ingredient may be immersed in a solution containing, as the natural antimicrobial, nisin, which is a bacteriocin, or a nisin-containing fermentation extract, and the surfactant, such as polylysine, vitamin B1 lauryl sulfate, and the like, in an amount of 0.01 to 1.0 weight%.

The immersion in the natural antimicrobial solution in combination with the pretreating microbial reduction step of microwave heating may reduce the initial microorganism count in the original ingredient, and thus, even milder retort condition treatment may sterilize microorganisms more effectively and preserve the organoleptic quality and nutritive components of the original ingredient.

In addition, in the sterilization method of the present application, the original ingredient may optionally be packaged together with sauce or broth in step 2). The sauce or broth may be prepared in a separate sauce or broth preparation step. Specifically, the sauce or broth may be prepared by heating and stirring materials for the sauce or broth, which and the contents of which have been determined depending on a desired product, at 85-95°C for 10 to 30 minutes, but the present application is not limited thereto.

The sauce or broth may be pretreated by microwave heating treatment. The microwave heating treatment may be the same as the above-mentioned microbial reduction treatment of the original ingredient, but detailed conditions for pretreatment may be changed depending on the characteristics of the sauce or broth.

The processed food of the present application may be for room temperature distribution. The sterilization method of the present application minimizes the heat-induced deterioration of qualities, such as inherent taste, flavor and texture, of raw materials of the processed food, compared to conventional sterilization methods, and therefore, the processed food sterilized by the above-mentioned sterilization method keeps the intrinsic textures of the vegetable, meat and seafood and the flavors of aromatic vegetables, such as red pepper, garlic, ginger, etc. considerably. The above-mentioned sterilization method may effectively sterilize fungi, yeast, pathogenic bacteria, and sporogenic heat-resistant microorganisms associated with hygiene and quality deterioration in raw materials, and thus, the processed food may be distributable at room temperature.

Hereinafter, the present application will be described in more detail through Examples. However, the contents described in Examples is merely an example of the present application, and the scope of the present application is not limited to the scope of Examples.

### [Examples]

### Preparation Example 1: Microwave heating pretreatment microbial reduction and mild retort-applied vegetable, meat, seafood and sauce (broth) based processed food preparation

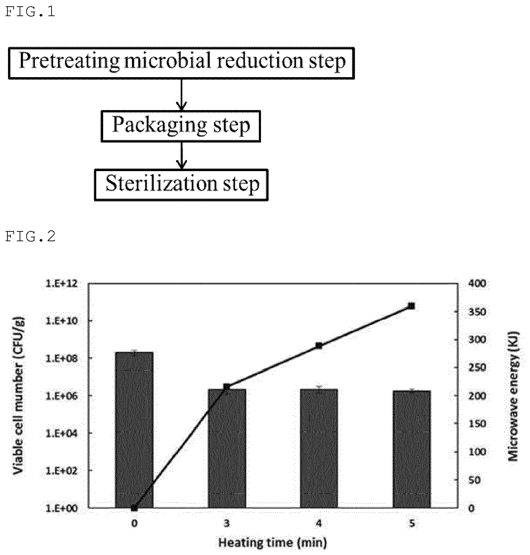

The representative preparation process for the vegetable, meat, seafood, and sauce or broth based processed food to which the microwave heating pretreatment microbial reduction and the mild retort fusion sterilization method of the present application is applied is as follows, and the process chart thereof is shown in FIG. 1.

### (1) Original ingredient raw material selecting, washing, and cutting process

Preparation of the raw material is carried out by washing the original ingredient with clean water approximately three times, in order to remove contaminants such as dirt, foreign substances, etc. attached to the surface of vegetables, meats, seafood, and then cutting the same into a size suitable for a food characteristic.

### (2) Pretreating microbial reduction process - immersion in the natural antimicrobial solution

Each of the vegetable, meat, and seafood raw materials that have been selected, washed and cut, is immersed in the natural antimicrobial solution of 0.01 to 3.0 (w/w, %) based on the mass of water for 30 to 120 minutes, and then is washed to be used. The natural antimicrobial used herein is an organic acid, a surfactant, a bacteriocin, a calcium preparation, and the like. The natural antimicrobial may be used alone or in combination, considering the organoleptic characteristics of the original ingredient, the microbial status, and the like. If there are few native bacteria such as heat-resistant bacteria in the raw material, the step of immersing in the natural antimicrobial solution may be omitted and the microwave preheating microbial reduction process may be applied immediately.

### (3) Pretreating microbial reduction process - microwave heating

The vegetable, meat, and seafood original ingredient raw materials which have been immersed in the natural antimicrobial solution are passed through a continuous or batch type microwave heating device to perform high-temperature, short-time heating sterilization. Microwave heating time may be selectively applied for between 2 and 10 minutes at normal pressure in consideration of organoleptic characteristics of the original ingredient and microorganism characteristics. In order to improve the sterilization effect, heating sterilization may be carried out under pressurized condition. In addition, the microwave heating pretreatment process has an advantage that the water or steam blanching effect may be obtained in addition to the sterilization effect.

In addition to vegetable, meat, and seafood original ingredients, the sauce or broth may also be sterilized continuously by using microwave heating equipment.

### (4) Sauce or broth preparation process

The sauce or broth are prepared by weighing raw materials, mixing the same and heating the same at 85 to 95°C at 10 to 30 minutes. In case of the sauce or broth which uses a lot of raw materials such as aromatic vegetables, a lot of heat-resistant bacteria grow naturally in the raw materials, and thus, such sauce or broth may be selectively passed through microwave heating sterilizing equipment to perform pretreating microbial reduction.

### (5) Original ingredient and sauce or broth packaging process

The microwave heating pretreatment microbial reduced vegetable, meat, seafood original ingredient and sauce or broth are quantified in consideration of a food characteristics, are put into a pouch or tray, and the pouch or tray is sealed and packaged. Checking the weight and foreign substances, followed by packaging may be carried out by using automatic rotary packer equipment for the pouch, or a tray automatic packaging machine for the tray.

### (6) Mild retort sterilization process

The finished product packaged in the pouch or tray is heat-sterilized at 110 to 121°C for 10 to 40 minutes by using hydrothermal or spray retort equipment. Herein, since the immersion in the natural antimicrobial and the pretreating microbial reduction using microwave heating have been applied previously, the heat sterilization condition which is reduced by around 50% compared to conventional retort conditions may be applied. Specifically, low temperature retort sterilization of heating at 110 to 115°C for 20 to 40 minutes or short time retort sterilization of heating at 121 to 125°C for 5 to 15 minutes, preferably for 7 to 12 minutes may be carried out.

Experimental example 1: Measurement of microwave heating pretreatment microbial reduction and mild retort combination sterilization effect on microorganisms in the original ingredient

Each of vegetable, meat, and seafood samples was treated with microwave heating pretreatment microbial reduction for 2 to 10 minutes and the sterilization effects on total bacteria, heat-resistant Bacillus bacteria, and fungi, such as fungi and yeast, respectively, were studied.

### (1) Measurement of the sterilization effect of microwave heating on vegetables

The effect of microwave heating sterilization on heat-resistant Bacillus bacteria, which is the main target bacteria of sterilization in room temperature distribution products, was examined. As a result, heating for at least 3 minutes resulted in around 10² (CFU/g) of *Bacillus amyloliquenfaciens* as shown in FIG. 2 and thus, the microwave heating sterilization exhibited the microbial reduction effect. Typically, the count of sporogenic microorganisms in the processed food is 10² to 10³ (CFU/g), and thus, microwave heating which showed the microbial reduction effect of about 10² (CFU/g) may be applied as a commercial pretreatment sterilization method.

### (2) Measurement of the effect of microwave heating and mild retort combination sterilization on total bacteria, heat-resistant bacteria, and fungi in vegetables

The microbial reduction effect and the sterilization effect of the microwave heating alone and microwave heating in combination with mild retort on the vegetable original ingredients treated in Preparation example 1 were examined and the results are shown in Table 1 compared to conventional heating retort sterilization effect.

**Table 1**

| The result of microwave heating and mild retort combination sterilization against total bacteria, heat-resistant bacteria, and fungi in vegetables | | | | | | | | |
|---|---|---|---|---|---|---|---|---|
| Samp les | Detailed conditions | Total bacteria (CFU/g) | | Heat-resistant bacteria (CFU/g) | | Fungi (CFU/g) | | Ov er al 1 ta st e (5 po in ts ) |
| | | Before sterili zation | After sterili zation | Before sterili zation | After sterili zation | Before sterili zation | After sterili zation | |
| Wash ed pota to (wit h peel ) 150 g | Control (121°C, 20 minutes) | 2×10⁶ | 0 | 4×10¹ | 0 | 5×10² | 0 | 3. 6^{a} |
| | Microwave heating (5 minutes) | 2×10⁶ | 4×10² | 4×10¹ | 0 | 5×10² | 0 | - |
| | Microwave heating (5 minutes) + mild retort (121°C, 10 minutes) | 2×10⁶ | 0 | 4×10¹ | 0 | 5×10² | 0 | 3. 9^{b} |
| Wash ed swee t pota to (wit h peel ) 150 g | Control (121°C, 20 minutes) | 5×10⁶ | 0 | 2×10² | 0 | 3×10² | 0 | 3. 7^{a} |
| | Microwave heating (3 minutes) | 5×10⁶ | 6×10² | 2×10² | 1×10¹ | 3×10² | 0 | - |
| | Microwave heating (3 minutes) + mild retort (121°C, 10 minutes) | 5×10⁶ | 0 | 2×10² | 0 | 3×10² | 0 | 4. 0^{b} |
| Swee t pump kin (Wit h rind ) 150 g | Control (121°C, 20 minutes) | 3×10⁶ | 0 | 1×10¹ | 0 | 2×10² | 0 | 3. 5^{a} |
| | Microwave heating (3 minutes) | 3×10⁶ | 2×10² | 1×10¹ | 0 | 2×10² | 0 | - |
| | Microwave heating (3 minutes) + mild retort (121°C, 10 minutes) | 3×10⁶ | 0 | 1×10¹ | 0 | 2×10² | 0 | 3. 9^{b} |

| | | | | | | | | |
|---|---|---|---|---|---|---|---|---|
| * Significant difference test (P < 0.05), values having the same English letter are not significantly different from each other. | | | | | | | | |

When potato, sweet potato, and sweet pumpkin, which are representative samples of vegetables, were treated with the microwave heating treatment alone, the count of total bacteria was reduced to around 10⁴ (CFU/g) and the count of heat-resistant bacteria was reduced to around 10¹ (CFU/g), and all fungi were killed. When the microwave heating pretreatment microbial reduction, followed by mild retort was applied, all microorganisms were killed. In addition, the microwave pretreatment and mild retort treatment resulted in the higher overall taste score compared to the conventional retort treatment. Therefore, it could be confirmed that the use of the sterilization method of the present application may reduce the organoleptic quality impairment due to the high-temperature heating compared to the conventional retort method, thereby improving the quality of the room temperature product. It may be said that this is a differentiating factor of the present application.

### (3) Measurement of the effect of microwave heating and mild retort combination sterilization on total bacteria, heat-resistant bacteria, and fungi in meats and seafood

The microbial reduction effect and the sterilization effect of the microwave heating alone and microwave heating in combination with mild retort on the meat and seafood original ingredients treated in Preparation example 1 were examined and the results are shown in Table 2 compared to conventional heating retort sterilization effect.

**Table 2**

| The result of microwave heating and mild retort combination sterilization against total bacteria, heat-resistant bacteria, and fungi in meats and seafood | | | | | | | | |
|---|---|---|---|---|---|---|---|---|
| Sampl es | Detailed conditions | Total bacteria (CFU/g) | | Heat-resistant bacteria (CFU/g) | | Fungi (CFU/g) | | Ov er al 1 ta st e (5 po in ts ) |
| | | Before sterili zation | After sterili zation | Before sterili zation | After sterili zation | Before sterili zation | After sterili zation | |
| Chick en (Raw) 150g | Control (121°C, 20 minutes) | 1×10⁴ | 0 | 2×10¹ | 0 | 2×10² | 0 | 3. 6^{a} |
| | Microwave heating (3 minutes) | 1×10⁴ | 2×10¹ | 2×10¹ | 0 | 2×10² | 0 | - |
| | Microwave heating (3 minutes) + mild retort (121°C, 10 minutes) | 1×10⁴ | 0 | 2×10¹ | 0 | 2×10² | 0 | 3. 9^{b} |
| Beef (Raw) 150 g | Control (121°C, 20 minutes) | 9×10³ | 0 | 4×10¹ | 0 | 5×10² | 0 | 3. 7^{a} |
| | Microwave heating (3 minutes) | 9×10⁶ | 1×10¹ | 4×10¹ | 0 | 5×10² | 0 | - |
| | Microwave heating (3 minutes) + mild retort (121°C, 10 minutes) | 9×10⁶ | 0 | 4×10¹ | 0 | 5×10² | 0 | 4. 0^{b} |
| Shrim ps (Peel ed shrim ps) 150 g | Control (121°C, 20 minutes) | 2×10⁴ | 0 | 1×10² | 0 | 3×10² | 0 | 3. 5^{a} |
| | Microwave heating (3 minutes) | 2×10⁴ | 3×10¹ | 1×10² | 1×10¹ | 3×10² | 0 | - |
| | Microwave heating (3 minutes) + mild retort (121°C, 10 minutes) | 2×10⁴ | 0 | 1×10² | 0 | 3×10² | 0 | 3. 8^{b} |

| | | | | | | | | |
|---|---|---|---|---|---|---|---|---|
| * Significant difference test (P < 0.05), values having the same English letter are not significantly different from each other. | | | | | | | | |

When chicken, beef, and shrimps, which are representative samples of meats and seafoods, were treated with the microwave heating treatment alone, the count of total bacteria was reduced to around 10³ (CFU/g) and the count of heat-resistant bacteria was reduced to around 10¹ (CFU/g), and all fungi were killed. When the microwave heating pretreatment microbial reduction, followed by mild retort of the present application was applied, all microorganisms were killed. In addition, the microwave pretreatment and mild retort treatment resulted in the higher overall taste score compared to the conventional retort treatment. Therefore, it could be confirmed that the use of the sterilization method of the present application may reduce the organoleptic quality impairment due to the high-temperature heating compared to the conventional retort method, thereby improving the quality of the room temperature product. It may be said that this is a differentiating factor of the present application.

From these results, it was concluded that appropriate combination of the microwave heating and mild retort may sterilize various native microorganisms and contaminating microorganisms in food materials such as vegetables, meats, and seafoods, while improving the quality. Accordingly, the microwave heating sterilization method of the present application could be used as a pretreatment sterilization technique.

Experimental example 2: Measurement of the sterilization effect of the combination with the natural antimicrobial immersion pretreating microbial reduction

The microbial reduction effect and the sterilization effect on vegetable based sauce (red pepper paste sauce) and meat based soup (cream soup) treated with the immersion in the natural antimicrobial, the microwave heating pretreatment microbial reduction, and mild retort in Preparation example 1 were observed in each treatment step and the results are shown in Table 3 compared to conventional heating retort sterilization effect.

**Table 3**

| The result of the sterilization of the combination with the natural antimicrobial immersion pretreating microbial reduction | | | | | | | | |
|---|---|---|---|---|---|---|---|---|
| Sa mp le s | Detailed conditions | Total bacteria (CFU/g) | | Heat-resistant bacteria (CFU/g) | | Fungi (CFU/g) | | Ov er al 1 ta st e (5 po in ts ) |
| | | Before sterili zation | After sterili zation | Before sterili zation | After sterili zation | Before sterili zation | After sterili zation | |
| Re d pe pp er pa st e sa uc e (1 50 g) | Control (121°C, 20 minutes) | 1×10⁶ | 0 | 3×10⁴ | 0 | 5×10⁴ | 0 | 3. 6^{a} |
| | Organic acid based natural antimicrobial (1%, 60 minutes immersion) | 1×10⁶ | 3×10⁴ | 3×10⁴ | 6×10³ | 5×10⁴ | 4×10³ | - |
| | Organic acid natural antimicrobial (1 weight%, 60 minutes immersion) + Microwave heating (3 minutes) | 1×10⁶ | 5×10¹ | 3×10⁴ | 4×10² | 5×10⁴ | 0 | - |
| | Organic acid natural antimicrobial (1 weight%, 60 minutes immersion) + Microwave heating (3 minutes) + mild retort (121°C, 10 minutes) | 1×10⁶ | 0 | 3×10⁴ | 0 | 5×10⁴ | 0 | 3. 9^{b} |
| Cr ea m so up | Control (121°C, 20 minutes) | 1×10⁵ | 0 | 7×10³ | 0 | 3×10³ | 0 | 3. 7^{a} |
| | Organic acid based natural antimicrobial (1 weight%, 60 | 1×10⁵ | 8×10³ | 7×10³ | 8×10² | 3×10³ | 7×10² | - |
| (1 50 g) | minutes immersion) | | | | | | | |
| | Organic acid natural antimicrobial (1 weight%, 60 minutes immersion) + Microwave heating (3 minutes) | 1×10⁵ | 2×10¹ | 7×10³ | 5×10¹ | 3×10³ | 0 | - |
| | Organic acid natural antimicrobial (1 weight%, 60 minutes immersion) + Microwave heating (3 minutes) + mild retort (121°C, 10 minutes) | 1×10⁵ | 0 | 7×10³ | 0 | 3×10³ | 0 | 4. 0^{b} |

| | | | | | | | | |
|---|---|---|---|---|---|---|---|---|
| * Significant difference test (P < 0.05), values having the same English letter are not significantly different from each other. | | | | | | | | |

As shown in Table 3, the immersion in 1 weight% organic acid based natural antimicrobial for 60 minutes resulted in the reduction of the counts of total bacteria and heat-resistant bacteria to around 10¹ - 10² (CFU/g). Accordingly, it is expected that the conditions of microwave heating and mild retort may be lessened, and thus, the quality may be further improved. In addition, the microwave pretreatment and mild retort treatment of the natural antimicrobial treated original ingredients resulted in the higher overall taste score compared to the conventional retort treatment. Therefore, it can be confirmed that even if the step of immersing the original ingredient in the natural antimicrobial is added, the quality of the room temperature product may be improved compared to the conventional retort treatment.

From the above research results, it can be seen that when the immersion in the natural antimicrobial and the microwave heating pretreatment microbial reduction and mild heat-sterilization method were fused and applied, the level of microorganisms such as nutrient cells, heat-resistant spores and fungi in vegetable, meat, seafood original ingredient based processed foods reaches sterilization, and thus, the same sterilization effect may be obtained as that of the retort method, which is a conventional high-temperature, high-pressure sterilization method, and the intensity of the retort heat-sterilization may be reduced by around 50% and accordingly, the processed food sterilization technology of the present application is a useful technology that may be utilized for the development of differentiated room temperature products in the future.

## Claims

1. A processed food sterilization method, comprising: 1) a pretreating microbial reduction step for microwave heat-treating an original ingredient; 2) a packaging step for packaging the pretreated original ingredient; and 3) a sterilization step for retort sterilizing the packaged original ingredient;
wherein the original ingredient in step 1) is an original ingredient having been immersed in a natural antimicrobial solution;
wherein in step 2), the original ingredient is packaged together with sauce or broth; and
wherein the retort sterilization in step 3) is performed for 20 to 40 minutes at 110 to 115°C, or the retort sterilization in step 3) is performed for 5 to 15 minutes at 121 to 125°C.

2. The processed food sterilization method of claim 1, wherein step 1) is performed under the condition of 133.322 - 226.6481 kPa (1,000 to 1,700 mmHg).

3. The processed food sterilization method of claim 1, wherein step 1) is microwave heating treatment for 1 minute to 30 minutes.

4. The processed food sterilization method of claim 1, wherein the natural antimicrobial solution comprises any one or more natural antimicrobials among organic acids, surfactants, bacteriocin, and calcium preparations.

5. The processed food sterilization method of claim 1, wherein the natural antimicrobial solution comprises 0.01 to 3.0 weight% of the natural antimicrobial.

6. The processed food sterilization method of claim 1, wherein the sauce or broth has been pretreated by microwave heating.

7. The processed food sterilization method of claim 1, wherein the processed food is for room temperature distribution.

## Patentansprüche

1. Verfahren zur Sterilisation von verarbeiteten Lebensmitteln, die Folgendes beinhaltet: 1) einen Vorbehandlungs-Keimreduzierungsschritt zur Mikrowellen-Wärmebehandlung einer Ausgangszutat; 2) einen Verpackungsschritt zur Verpackung der vorbehandelten Ausgangszutat; und 3) einen Sterilisierungsschritt zur Sterilisation der verpackten Ausgangszutat im Autoklaven;
wobei die Ausgangszutat in Schritt 1) eine Ausgangszutat ist, die in eine natürliche antimikrobielle Lösung getaucht wurde;
wobei in Schritt 2) die Ausgangszutat zusammen mit Sauce oder Brühe verpackt wird; und
wobei die Sterilisation im Autoklaven in Schritt 3) 20 bis 40 Minuten lang bei 110 bis 115 °C durchgeführt wird oder die Sterilisation im Autoklaven in Schritt 3) 5 bis 15 Minuten lang bei 121 bis 125 °C durchgeführt wird.

2. Verfahren zur Sterilisation von verarbeiteten Lebensmitteln nach Anspruch 1, wobei Schritt 1) unter der Bedingung von 133,322 - 226,6481 kPa (1000 bis 1700 mmHg) durchgeführt wird.

3. Verfahren zur Sterilisation von verarbeiteten Lebensmitteln nach Anspruch 1, wobei Schritt 1) eine Mikrowellenerwärmungsbehandlung für 1 Minute bis 30 Minuten ist.

4. Verfahren zur Sterilisation von verarbeiteten Lebensmitteln nach Anspruch 1, wobei die natürliche antimikrobielle Lösung ein oder mehr natürliche antimikrobielle Substanzen unter organischen Säuren, Tensiden, Bakteriocin und Kalziumpräparaten beinhaltet.

5. Verfahren zur Sterilisation von verarbeiteten Lebensmitteln nach Anspruch 1, wobei die natürliche antimikrobielle Lösung 0,01 bis 3,0 Gewichts-% der natürlichen antimikrobiellen Substanz beinhaltet.

6. Verfahren zur Sterilisation von verarbeiteten Lebensmitteln nach Anspruch 1, wobei die Sauce oder Brühe durch Mikrowellenerwärmung vorbehandelt wurde.

7. Verfahren zur Sterilisation von verarbeiteten Lebensmitteln nach Anspruch 1, wobei das verarbeitete Lebensmittel für den Vertrieb bei Raumtemperatur bestimmt ist.

## Revendications

1. Procédé de stérilisation d'aliments transformés, comprenant : 1) une étape de réduction microbienne prétraitée pour traiter thermiquement par micro-ondes un ingrédient original ; 2) une étape de conditionnement pour conditionner l'ingrédient original prétraité ; et 3) une étape de stérilisation pour stériliser en cornue l'ingrédient original conditionné ;
dans lequel l'ingrédient original à l'étape 1) est un ingrédient original ayant été immergé dans une solution antimicrobienne naturelle ;
dans lequel à l'étape 2), l'ingrédient original est conditionné avec une sauce ou un bouillon ; et
dans lequel la stérilisation en cornue à l'étape 3) est effectuée pendant de 20 à 40 minutes à de 110 à 115 °C, ou la stérilisation en cornue à l'étape 3) est effectuée pendant de 5 à 15 minutes à de 121 à 125 °C.

2. Procédé de stérilisation d'aliments transformés selon la revendication 1, dans lequel l'étape 1) est effectuée dans des conditions de 133,322 - 226,6481 kPa (1.000 à 1.700 mmHg).

3. Procédé de stérilisation d'aliments transformés selon la revendication 1, dans lequel l'étape 1) est un traitement thermique par micro-ondes pendant de 1 minute à 30 minutes.

4. Procédé de stérilisation d'aliments transformés selon la revendication 1, dans lequel la solution antimicrobienne naturelle comprend un quelconque ou plusieurs agents antimicrobiens naturels parmi des acides organiques, des tensioactifs, des préparations à base de bactériocines et de calcium.

5. Procédé de stérilisation d'aliments transformés selon la revendication 1, dans lequel la solution antimicrobienne naturelle comprend de 0,01 % à 3,0 % en poids de l'agent antimicrobien naturel.

6. Procédé de stérilisation d'aliments transformés selon la revendication 1, dans lequel la sauce ou le bouillon a été prétraité par chauffage micro-ondes.

7. Procédé de stérilisation d'aliments transformés selon la revendication 1, dans lequel l'aliment transformé est destiné à la distribution à température ambiante.
